# EUROPÄISCHE PATENTANMELDUNG

(11) **EP 4 480 930 A1**
(43) Veröffentlichungstag der Anmeldung: **25.12.2024**
(21) Anmeldenummer: 24181977.0
(22) Anmeldetag: 13.06.2024
(51) Int. Cl.: C02F 3/32, C02F 3/10, C12M 1/00

(54) **MIKROALGENBIOFILMREAKTOR**

(30) Priorität: 20.06.2023 DE 102023116102
(71) Anmelder: Forschungszentrum Jülich GmbH, 52425 Jülich (DE)
(72) Erfinder: Dr. Reinecke-Levi, Diana, 52428 Jülich (DE)
(74) Vertreter: Meissner Bolte Düsseldorf Patentanwälte Rechtsanwälte Partnerschaft mbB

(57) **Zusammenfassung**

Die vorliegende Erfindung betrifft eine Vorrichtung zur Aufarbeitung von Abwasser, bei der das Abwasser Kultivierungsflächen durchströmt, wobei die Kultivierungsflächen eine jeweilige Neigung in Bezug auf die Horizontale zwischen 0,5° und 7,5° aufweisen und die Aufarbeitung in einem Gehäuse erfolgt, sowie entsprechende Verfahren zur Abwasseraufbereitung und Verwendungen.

## Beschreibung

Alle in der vorliegenden Anmeldung zitierten Dokumente sind durch Verweis vollumfänglich in die vorliegende Offenbarung einbezogen (= incorporated by reference in their entirety).

Die vorliegende Erfindung betrifft Vorrichtungen zur Aufbereitung von Abwasser, insbesondere für die Phytoremediation von Abwässern (landwirtschaftlichen, industriellen, kommunalen, Aquakulturen) mittels eines erntefähigen stationären Algenbiofilms.

### Stand der Technik:

Die Kultivierung von eukaryotischen und prokaryotischen Mikroalgen für Forschungszwecke und biotechnologische Anwendungen hat zu der Etablierung von zahlreichen Anwendungsfeldern und entsprechenden Reaktortypen geführt. Die Fähigkeiten von Mikroalgen verschiedenste Nähr- und Schadstoffe anzureichern und/oder abzubauen findet bereits im begrenzten Umfang in der Remediation von Abwässern und Abgasen statt. Nachteilig ist jedoch, dass die meisten Anwendungen stark auf Monokulturen und suspendierte Systeme fokussieren. Dies schränkt die techno-ökonomische Machbarkeit von vielen Anwendungen stark ein. Ein alternativer Ansatz ist hier die Kultivierung von gemischten (mesokosmischen) Algen-Biofilmen, welche eine höhere metabolische Flexibilität aufweisen bei gleichzeitig geringeren techno-ökonomischen Aufwendungen.

Bekannte Algal Turf Scrubber (ATS) Systeme, wie zum Beispiel in WO 96/13970 A1 und US 4,333,263 beschrieben, sind offen zur Umwelt und können zur Freisetzung von potenziell schädlichen Abwässern und Biofilm führen. Kommerziell verfügbare Algen-Biofilm-Systeme wie in WO 2014/172691 A1 nutzen eine mobile Kultivierungsfläche, die über Umlenkrollen bewegt wird. Dieses und vergleichbare Systeme wie in WO 2014/085869 A1 weisen nachteilige, hohe Energieverbräuche, technische Ausfallraten und Aufwendungen, sowie Biofilmablösungen auf. Umschlossene Systeme wie in DE 20 2019 005 811 U1 eigenen sich nur für kleinste Abwassermengen und sind für den Einsatz im großtechnischen Maßstab ungeeignet.

Ein weiteres Dokument des Standes der Technik ist D. Reinecke et al., "Nutrient recovery from wastewater by algal biofilm fertilizer production part 1: Case study on the techno-economical aspects at pilot-scale", Separation and Purification technology 305 (2023) 122471.

Zudem können als weitere Dokumente des Standes der Technik WO 2024/038269 A1 (worin entweder Suspensionen/Lösungen von Mikroorganismen oder auf wässrigem Strom schwimmende Mikroorganismen verwendet werden), DE 10 2014 000 691 A1 (betreffend im Kreislauf geführte Suspensionen freischwimmender Mikroorganismen), US 2007/0155006 A1 (betreffend die Kultivierung von Mikroalgen in Suspensionen), WO 2015/087169 A2 (betreffend eine Vielzahl von Kulturreaktoren für Mikroorganismen in dedizierten Gestellen), sowie DE 10 2009 016 738 A1 (betreffend spezielle Reaktorsysteme für Kultursuspensionen) genannt werden. Diese fünf Dokumente betreffen Prozessführungen basierend auf freischwimmenden Mikroorganismen im Kreislauf, die unter anderem einen hohen Ressourcenaufwand bei der Ernte erfordern (Zentrifugation, Filtration). Die beiden letztgenannten sind geschlossene Systeme für hochpreisige Produkte.

Offene Systeme weisen je nach Umgebungstemperatur eine hohe Verdunstungsrate und Freisetzung von unangenehmen Gerüchen und potenziell schädlichem Gasen (NH₃, VOC, CH₄) auf.

Daher wird nach wie vor nach weiteren Methoden gesucht, die es ermöglichen, Abwässer auf einfache, zuverlässige und effiziente Art und Weise aufzubereiten.

Aufgabe der vorliegenden Erfindung war es daher im Hinblick auf den Stand der Technik neue Vorrichtungen zur Aufarbeitung von Abwässern zur Verfügung zu stellen, die gegenüber den Vorrichtungen des Standes der Technik verbessert sind und neue bzw. weitere Vorteile aufweisen.

Weitere Aufgabenstellungen ergeben sich für den Fachmann aus der nachfolgenden Beschreibung.

Gelöst werden diese und andere Aufgaben im Rahmen der vorliegenden Erfindung durch die Gegenstände der unabhängigen Ansprüche.

Bevorzugte Ausgestaltungen ergeben sich aus den abhängigen Ansprüchen sowie der nachfolgenden Beschreibung.

Im Rahmen der vorliegenden Erfindung schließt der Begriff «umfassen» jeweils als besonders bevorzugte Ausgestaltung auch «bestehend aus» ein; das heißt eine entsprechende Liste kann neben den explizit genannten Elementen auch weitere Elemente enthalten (= umfassen), oder sie kann genau diese Elemente enthalten (=bestehen aus) (wobei unwesentliche Elemente wie Schrauben, Markierungen etc. nicht berücksichtig sind).

Bei relativen Angaben wie oben, unten, links, rechts oder Ähnlichem wird im Rahmen der vorliegenden Erfindung als Bezugssystem von einem aufrecht auf dem Erdboden vor dem diskutierten Objekt stehenden Beobachter ausgegangen.

Im Rahmen der vorliegenden Erfindung bedeutet der Ausdruck "und/oder" dass beide in dem Zusammenhang genannten Elemente jeweils einzeln als auch die Kombination der in dem Zusammenhang genannten Elemente umfasst ist.

Im Rahmen der vorliegenden Erfindung sind alle Mengenangaben, sofern nicht anders angegeben, als Gewichtsangaben zu verstehen.

Im Rahmen der vorliegenden Erfindung bedeutet der Begriff "Umgebungstemperatur" eine Temperatur von 20°C. Temperaturangaben sind, soweit nicht anders angegeben, in Grad Celsius (°C).

Sofern nichts Anderes angegeben wird, werden die angeführten Reaktionen bzw. Verfahrensschritte bei Atmosphärendruck, d.h. bei etwa 1013 kPa durchgeführt. Wenn im Rahmen der vorliegenden Erfindung von "Ernte" gesprochen wird, so ist damit gemeint, dass die Biomasse bzw. der (Algen-)Biofilm von der Kultivierungsfläche entfernt, zum Beispiel abgeschabt, wird; das so erhaltene Produkt kann dann als Biodünger, Biogassubstrat, Bodenkonditionierer, Bodenzuschlagstoff oder ähnliches verwertet werden.

Die vorliegende Erfindung betrifft in einem ersten Gegenstand eine Vorrichtung zur Aufarbeitung von Abwasser welche alle entsprechend notwendigen Vorrichtungen bzw. Einrichtungen in einem (einzigen) allseits geschlossenen Gehäuse umfasst.

Der Innenraum des Gehäuses ist im Rahmen der vorliegenden Erfindung bevorzugt aus korrosionsbeständigem und abwaschbarem Material oder ist mit einem solchen beschichtet; ein Beispiel für bevorzugt verwendbare Materialien ist Edelstahl. Denn naturgemäß herrscht im Betrieb der erfindungsgemäßen Vorrichtung im Innenraum eine gewisse Luftfeuchtigkeit. Diese kann sich an den Wänden niederschlagen und könnte - bei anderen Materialien - dort zu Korrosion führen. Zudem ist es gefährlich für das Bedienungspersonal, dass zum Beispiel zu Wartungszwecken den Innenraum betritt, wenn sich auf dem Boden Feuchtigkeit sammelt, die aber nicht weggewischt werden kann. Schließlich beruht die vorliegende Erfindung auf biologischen Prozessen. Insofern ist es sinnvoll Verunreinigungen von den Oberflächen (des Gehäuses) entfernen zu können (diese könnte ja auch zu Verunreinigungen des gereinigten Wassers führen).

In besonders bevorzugten Ausgestaltungen der vorliegenden Erfindung ist das Gehäuse ein quaderförmiges Gehäuse, bevorzugt ein, gegebenenfalls umgebauter, Container, besonders bevorzugt ein, gegebenenfalls umgebauter, Standardcontainer oder ISO-Container, insbesondere ein, gegebenenfalls umgebauter, 10-Fuß-Schiffscontainer, 20-Fuß-Schiffscontainer oder 40-Fuß-Schiffscontainer. Insbesondere ermöglicht solch ein Container, bevorzugt durch Transportösen und/oder Anbringung von Transversen und Standfüßen, den Transport und mobilen Einsatz.

Das allseits geschlossene Gehäuse weist ein oder mehr öffen- und schließbare Zugänge auf. Dies können im Prinzip alle Arten von Zugängen sein, also zum Beispiel Mannlöcher, Luken, Türen etc. bevorzugt sind dies Türen, die entweder in die jeweiligen Kopfseiten des Gehäuses eingearbeitet sind oder aber die ganzen jeweiligen Kopfseiten ausbilden; letzteres ist oft der Fall bei ISO-Containern, welche eine bevorzugte Art von Gehäuse im Rahmen der vorliegenden Erfindung sind.

Dazu sind im Inneren des Gehäuses zwei oder mehr Kultivierungsflächen (technisch wissenschaftlich auch als "Flowway" bezeichnet) übereinander angeordnet. Diese sind dabei bevorzugt parallel zueinander angeordnet, d.h. insbesondere, dass die durch ihre Grundflächen gebildeten Ebenen parallel zueinander sind. Ferner weisen die Kultivierungsflächen (bzw. die durch ihre Grundflächen gebildeten Ebenen) jeweils eine Neigung in Bezug auf die Horizontale zwischen 0.5° und 7,5°, bevorzugt zwischen 1° und 5°, besonders bevorzugt zwischen 1,5° und 3,5° und insbesondere 2°, auf, wobei als Horizontale die Grundfläche des Gehäuses (Boden des Containers) gilt.

Die einzelnen Kultivierungsflächen können dabei, in Bezug auf das Gehäuse im Prinzip in beliebiger Art und Weise geneigt sein, zum Beispiel auch schräg innerhalb des Gehäuses, bevorzugt ist es aber, wenn sie derart geneigt sind, das sie, in einer Schnittansicht des Gehäuses von einer Seite des Gehäuses zur gegenüberliegenden Seite geneigt sind, wobei es nicht erforderlich und in der Regel auch nicht so ausgeführt ist, dass die Enden der Kultivierungsflächen an die Seitenwände anstoßen. Dort, am Ein- und Auslassende der Kultivierungsfläche, werden in bevorzugten Ausgestaltungen der vorliegenden Erfindung Zu- und Abführvorrichtungen für das Abwasser vorgesehen.

Unter "Kultivierungsfläche" werden im Rahmen der vorliegenden Erfindung flache, ebene Werkstücke verstanden, die längsseitige Ränder aufweisen, so dass diese einen nach einer Seite (oben) offenen Raum mit einem nutzbaren Volumen bilden, wobei Raum und Volumen durch die Bodenfläche, die Seitenlängen und die niedrigste Höhe der Ränder gebildet werden, und die dafür vorgesehen sind, Biofilme, sowie gegebenenfalls Bewuchshilfen (technisch wissenschaftlich auch als "Substrat" bezeichnet) für die Biofilme bzw. die die Biofilme bildenden Organismen, in das entstandene Volumen aufzunehmen. Die längsseitigen Ränder sind in bevorzugten Ausgestaltungen zwischen 1 cm und 10 cm, besonders bevorzugt zwischen 2 cm und 5 cm, hoch. In Varianten der vorliegende Erfindung ist es möglich, wenn die Kopf- und Fußenden, also die Seite, auf der das aufzureinigende Wasser aufgegeben wird, und die Seite, von der das gereinigte Wasser abgeführt wird, höhere oder niedrigere oder sogar gar keine Ränder aufweisen - in Abhängigkeit davon, wie die Wasserzu- und -abführvorrichtungen ausgestaltet sind. Entsprechend ist es auch möglich Löcher in den Rändern vorzusehen. Die so gebildeten Werkstücke - Kultivierungsflächen - haben meist, bevorzugt, rechteckige Grundflächen; jedoch sind ebenso parallelogrammartige, trapezoide, dreieckige, n-eckige oder ellipsoide Grundflächen denkbar.

In einem 40-Fuß-Container können in einer bevorzugten Ausführungsform Kultivierungsflächen mit den Dimensionen 11500 mm x 1500 mm eingesetzt werden, deren Seiten bevorzugt um 90° hochgebogen sind und dann eine Höhe von 30 mm aufweisen.

Die Kultivierungsflächen sind im Rahmen der vorliegenden Erfindung insbesondere aus inerten, das heißt insbesondere korrosionsbeständigen, abwaschbaren, chemikalienbeständigen, und reflektierenden Materialien gefertigt. Ein Beispiel für ein solches Material ist Edelstahl.

Die Kultivierungsflächen sind in ihren Dimensionen im Prinzip nicht beschränkt, solange sie, und gegebenenfalls vorhandene weitere Vorrichtungen/Einrichtungen, Platz in Inneren des Gehäuses der erfindungsgemäßen Vorrichtung finden.

In einem 40-Fuß-Container zum Beispiel kann ein Stapel von zehn Kultivierungsflächen mit 11500 mm x 1500 mm x 30 mm (letzteres die Kantenhöhe) ohne weiteres an einer Containerhälfte angeordnet werden, während in der anderen Hälfte noch genügend Platz verbleibt, damit dieser begehbar ist; zum Beispiel als Wartungsarm. Der Abstand zwischen den einzelnen Kultivierungsflächen kann in dieser Ausführungsform zum Beispiel 250 mm betragen (Fläche zu Fläche, die Randhöhen nicht berücksichtigt).

Die Oberflächen der Kultivierungsflächen werden von anhaftenden Biofilmen "belegt" (rasenartig bewachsen), bevorzugt von Mikroalgen-Biofilmen, wobei die auf den unterschiedlichen Kultivierungsflächen anhaftenden Biofilme alle gleich, zum Teil gleich oder alle unterschiedlich sind, wobei "gleich" bevorzugt bedeutet, dass gleiche Mikroorganismen eingesetzt werden. Im wissenschaftlichen Kontextbezieht sich die Vergleichbarkeit auf Biofilme mit ähnlichem Aufbau und ähnlicher Funktion trotz differenzierter mikrobieller Zusammensetzung. Selbst bei gleichem Inokkulum können final abweichende Biofilme auf den Flowways (Kultivierungsflächen; dort fließt das Wasser) entstehen (mathematisch-dynamisches System); siehe auch D. Reinecke et al., Separation and Purification technology 305 (2023) 122471. Das bedeutet, dass bei zum Beispiel drei Kultivierungsflächen drei verschiedene Biofilme vorhanden sein können, also auf jeder Kultivierungsflächen ein anderer, oder es kann auf allen dreien jeweils der gleiche Biofilm aufbracht sein, oder es können auf zweien die gleichen Biofilme sein und auf der dritten Kultivierungsflächen ein anderer. Sinngemäß gilt dies für jede Anzahl von Kultivierungsflächen.

Wie dem Fachmann wohl bekannt ist, sind für diese Zwecke, also zur Herstellung eines Biofilms, verschiedene Organismen geeignet, die der Fachmann aufgrund seines allgemeinen Fachwissens auswählen kann. Die genaue Biofilmzusammensetzung variiert dabei mit den Kulturbedingungen (Abwasserqualität, Temperatur, Licht etc.). Im Laufe der Zeit wird sich bei gleichbleibenden Kulturbedingungen ein optimierter und selektierter = gezüchteter Biofilm bilden, so dass bei jeder erfindungsgemäßen Vorrichtung im laufenden Betrieb ein individueller/lokaler Biofilm kultiviert wird. Diese Eigenart von biologischen System beruht auf der Adaption an die jeweiligen Kulturbedingungen und kann zur Züchtung von effektiven Algen-Biofilmen genutzt werden. Zunächst (also bei der Erstbeschickung/Bepflanzung) einsetzbar sind zum Beispiel (aber nicht abschließend) folgende: Aus der Gruppierung der pro- und eukaryotischen Stämme: Mikroalgen (z.B. Spirulina, Chlorella); aus dem prokaryotischen Reich: Bakterien; aus den eukaryotischen Reichen: Metazoen (Wimpertierchen) und Pilze; Zusätzlich der Biofilm-Matrix: von den Mikroorganismen ausgeschiedene extrazelluläre polymere Substanzen (EPS).

Bevorzugt werden im Rahmen der vorliegenden Erfindung für die Biofilme vor Ort vorkommende Organismen eingesetzt (zum Beispiel Mikroalgen aus der lokalen Umgebung), wodurch die Umweltverträglichkeit gesteigert wird, denn dadurch wird zum Beispiel eine Kontamination der Umgebung durch vor Ort nicht ansässige Organismen vermieden (Stichworte Neophyten- bzw. Neomycetenvermeidung).

Wie dem Fachmann bekannt und daher nur zur Klarstellung nochmals ausgeführt, bedeutet die Formulierung "Kultivierungsflächen belegende Biofilme", dass die Biofilme, bevorzugt Mikroalgen-Biofilme, auf den Kultivierungsflächen direkt oder auf gegebenenfalls vorhandenem Aufwuchsmaterial für den Biofilm anhaften, also im Rahmen der üblichen Betriebsbedingungen der erfindungsgemäßen Vorrichtungen/Verfahren ortsfest sind und durch das die Kultivierungsflächen entlang fließende Wasser nicht abgelöst werden und insbesondere keine Suspensionen bilden (wobei einzelne bzw. in geringer Zahl abgelöste Mikroalgen, die suspendiert im fließenden Wasser weggeschwemmt werden, nicht als Suspension verstanden werden).

Weiterhin umfasst die erfindungsgemäße Vorrichtung mindestens eine Zuführvorrichtung zur Aufbringung von aufzuarbeitendem Abwasser auf ein oder mehrere Kultivierungsflächen. Dabei ist es erfindungsgemäß bevorzugt, wenn eine Vorrichtung das auf sie aufgegebene Abwasser auf alle Kultivierungsflächen verteilt. Allerdings ist es im Rahmen der vorliegenden Erfindung auch möglich, dass mehrere Zuführvorrichtungen zugegen sind, die dann entweder einzelne Kultivierungsflächen mit Abwasser beaufschlagen oder auch mehrere. In einigen bevorzugten Varianten der vorliegenden Erfindung wird das Abwasser durch eine Zuführvorrichtung gleichmäßig auf alle Kultivierungsflächen verteilt. Insbesondere ist die mindestens eine Zuführvorrichtung im Rahmen der vorliegenden Erfindung so angeordnet, dass das aufzuarbeitende Wasser auf das jeweils höhere Ende der jeweiligen - geneigten Kultivierungsflächen - aufgebracht wird; auf diese Art und Weise wird gewährleistet, dass das Abwasser durch die Schwerkraft getrieben die Kultivierungsflächen entlang fließt und somit die Biofilme durchläuft.

In bevorzugten Ausführungsformen der vorliegenden Erfindung umfasst die Zuführvorrichtung zur Aufbringung von aufzuarbeitendem Abwasser eine regelbare (justierbare) Pumpe für das Abwasser.

Ferner umfasst die erfindungsgemäße Vorrichtung noch mindestens eine Abführvorrichtungen für das aufgearbeitete Wasser, wobei die mindestens eine Abführvorrichtung so angeordnet ist, dass sie das aufgearbeitete Wasser von dem jeweils niedrigeren Ende der jeweiligen Kultivierungsflächen abführt. Sie bildet also quasi das Gegenstück zu der Zuführvorrichtung.

Die von der erfindungsgemäßen Vorrichtung umfassten mindestens eine Zuführvorrichtung und die mindestens eine Abführvorrichtung sind im einfachsten Falle einfache Rohre mit Abzweigungen zu den einzelnen Kultivierungsflächen. Bevorzugt ist es, wenn die Zuführvorrichtungen zur gleichmäßigen Verteilung auf die einzelnen Kultivierungsflächen angepasste Abzweigungen aufweisen, zum Beispiel unterschiedliche Durchmesser, oder entsprechende Regelvorrichtungen wie Ventile. In weiteren bevorzugten Ausgestaltungen der vorliegenden Erfindung weisen die Abzweigungen zu den einzelnen Kultivierungsflächen Verbreiterungen, zum Beispiel bis zu der gesamten Breite der jeweiligen Kultivierungsfläche, auf, um eine gleichmäßige Aufgabe des Wassers auf die jeweilige Kultivierungsfläche zu gewährleisten. Die Abführvorrichtungen weisen in einigen bevorzugten Varianten entsprechende Verbreiterungen auf, um das gereinigte Wasser aufzufangen. In anderen Varianten weisen die Kultivierungsflächen an ihren Enden ein oder mehrere Abflusslöcher auf, an die dann die Abführvorrichtung(en) angeschlossen ist (sind). Wenn die Abflusslöcher so ausgestaltet sind, dass sie ein Auffangen des Wassers an Boden des Gehäuses mittels eine Rinne oder Ähnlichem erlauben, dann sind die Abflusslöcher und die Rinne als Abführvorrichtung aufzufassen.

Die mindestens eine Zuführvorrichtung und die mindestens eine Abführvorrichtung sind bevorzugt, aber nicht zwingend, innerhalb des Gehäuses angeordnet, so dass der Transport der Vorrichtung einfacher ist, als wenn sie an der Außenseite angeordnet wären. Dies schließt in einigen bevorzugten Varianten der vorliegenden Erfindung zugehörige Vorrichtungen, wie Pumpen etc. ein.

In bevorzugten Varianten der vorliegenden Erfindung sind die mindestens eine Zuführvorrichtung und die mindestens eine Abführvorrichtung so ausgestaltet, dass sie mit genormten bzw. standardisierten Anschlüssen versehen sind, so dass das aufzuarbeitende Abwasser bzw. das gereinigte Wasser mittels üblicher Verbindungselemente vor Ort mit den vorhandenen Wassernetz oder ähnlichem verbunden werden können. Diese genormten bzw. standardisierten Anschlüsse können zum Beispiel entsprechend standardisierte Anschlüsse für Schläuche oder Rohrleitungen sein. Für den ordnungsgemäßen Betrieb der erfindungsgemäßen Vorrichtungen ist dies aber nicht in jedem Fall notwendig, da es auch möglich ist, aufzureinigendes Wasser einfach mittels beispielsweise einer Rinne oder eines Trichters in die Zuführvorrichtung zu geben und das aufgereinigte Wasser im einfachsten Fall einfach in die Umgebung ablaufen zu lassen (was meist aber Verschwendung wäre und daher in der Regel nicht erfolgt).

Unabhängig davon, wie genau die Zu- und Abführvorrichtungen ausgestaltet sind, ist es bevorzugt, wenn diese zumindest nach außen hin (d.h. gegenüber dem Äußeren des Gehäuses) verschließbar sind (was insbesondere für den Transport von Vorteil ist).

Weiterer Bestandteil der erfindungsgemäßen Vorrichtung ist mindestens eine Belüftungseinrichtung für das Gehäuse, konfiguriert Luft, insbesondere Umgebungsluft, zuzuführen und verbrauchte Luft abzuführen. In einigen bevorzugten Varianten der vorliegenden Erfindung ist dies eine passive Belüftungseinrichtung, insbesondere in Form von Belüftungslöchern, die in das Gehäuse eingearbeitet sind. In diesem Fall ist es bevorzugt, wenn eines oder mehrere Belüftungslöcher am Boden, oder in der Nähe des Bodens, des Gehäuses ausgestaltet sind, und eines oder mehrere in der Decke, oder in der Nähe der Decke, des Gehäuses. In anderen bevorzugten Ausführungsformen der vorliegenden Erfindung kommen aktive Belüftungseinrichtungen zum Einsatz, wie zum Beispiel Ventilatoren und/oder Gebläse. Vorteilhaft kann es sein, wenn an den Oberkanten der Öffnungen der Belüftungseinrichtungen zur Umgebung hin Abschirmungen angebracht sind, die zum Beispiel den Einfall von Regen verhindern. Im Falle von aktive Belüftungseinrichtungen ist es in Varianten bevorzugt, wenn die Luftansaugung über einen Filter oder ähnliches erfolgt.

Unabhängig davon, ob aktive oder passive Belüftungseinrichtungen eingesetzt werden, ist es bevorzugt, wenn diese zumindest nach außen hin (d.h. gegenüber dem Äußeren des Gehäuses) verschließbar sind (was insbesondere für den Transport von Vorteil ist).

Noch ein weiterer Bestandteil ist mindestens eine Einrichtung zur Zufuhr von Licht zu den Biofilmen, welche passiv oder aktiv sein kann. Diese sind bevorzugt so ausgestaltet, dass sie regelbar sind, d.h. dass das in das Gehäuse und insbesondere auf die Kultivierungsflächen gelangende Licht in seiner Menge und Intensität angepasst werden kann. Dies kann für die jeweils gewählte(n) Einrichtung(en) manuell oder auch elektronisch geregelt werden.

Die Einrichtungen zur Lichtzufuhr werden bevorzugt ausgewählt aus der Gruppe bestehend aus
a) lichtdurchlässigen, bevorzugt tageslichtdurchlässigen, Seitenelementen in mindestens einer, bevorzugt genau einer, Gehäusewandung (Längsseite des Gehäuses),
b) lichtdurchlässigen, bevorzugt tageslichtdurchlässigen, Elementen in der Gehäusedecke,
c) künstlichen Lichtquellen, bevorzugt Lampen, besonders bevorzugt LED, insbesondere bevorzugt innerhalb des Gehäuses angeordnet, insbesondere gegenüber a) liegend angeordnet, so dass die Kultivierungsflächen zwischen a) und c) angeordnet sind,

- und Kombinationen davon.

Von diesen ist die Variante b) weniger üblich (unter anderem weil es kaum Container gibt, die entsprechende Deckenausgestaltungen aufweisen und unter anderem weil die oberste Kultivierungsfläche abschattend für die darunterliegenden wirkt), aber dennoch genauso möglich, wie die Varianten a) und c).

Die Varianten a) und b) sind passive Einrichtungen zur Lichtzufuhr, da sie davon abhängen, ob und wenn ja wieviel Licht außerhalb des Gehäuses vorliegt bzw. erzeugt wird. Die Variante c) hingegen ist eine aktive Einrichtung, da von ihr (Energieversorgung vorausgesetzt) unabhängig von der Umgebung Licht erzeugt werden kann. Die Einrichtungen der Varianten a) und b) sind durch einfache Maßnahmen wie, insbesondere reflektierenden, Jalousien regelbar; sie können aber beispielsweise auch elektrisch regelbare Polarisationsfilter umfassen oder ähnliches.

Die Einrichtungen der Variante c) sind auf verschiedene Arten regelbar. So kann eine Lampen einfach per Dimmer geregelt werden. Für den Fall, dass mehrere einzelne Lampenelemente vorhanden sind, können diese auch jeweils unabhängig voneinander gedimmt werden, oder auch unabhängig voneinander an- oder ausgeschaltet werden. Sowohl die aktiven als auch die passiven Lichtzuführungseinrichtungen können durch Einsatz von Filtern modifiziert werden, so dass gegebenenfalls nur besondere Wellenlängen oder Wellenlängenbereiche des Lichts zu den Biofilmen gelangen.

Die Lampen können dabei in beliebiger Art und Weise positioniert sein bzw. werden; sie können zum Beispiel auf Ständern montiert sein, an den Wänden befestigt sein oder aufgehängt sein.

In besonders bevorzugten Ausgestaltungen sind die Lampen, insbesondere LED, nach Art eines Vorhang ausgestaltet, so dass sich die Lampen verschieben lassen. Dabei hängen die Lampen von der Gehäusedecke herab, und sind dabei so befestigt, dass sie einzeln oder in Gruppen verschoben werden können, insbesondere seitlich. Dies ist eine besonders flexible Lösung zur Zuführung von Licht, mittels derer sich das Licht einfach durch Verschieben an gewünschten Stellen intensivieren oder abschwächen lässt. In Ausführungsformen, bei denen dieser Lampen-Vorhang nicht den Gehäuseboden berührt, sondern darüber hängt, ist diese Lösung darüber hinaus auch aus Blickpunkten der einfacheren Reinigungsmöglichkeiten und Hygiene vorteilhaft.

In bevorzugten Varianten der vorliegenden Erfindung weist das Gehäuse mindestens ein lichtdurchlässiges, bevorzugt tageslichtdurchlässiges, Seitenelement in einer Gehäusewandung, und innerhalb des Gehäuses angeordnete Lampen, bevorzugt LED, insbesondere als Lampen-Vorhang, auf.

Dabei können verschiedenste, dem Fachmann wohlbekannte, Lampen eingesetzt werden. Die für die Beleuchtung von Pflanzen einsetzbar sind. Ein Beispiel für kommerziell erhältliche (vertikale) LED-Beleuchtungstechnologie im Pflanzenanbau sind Lampen, wie zum Beispiel von der Firma Blooms Grow Tech^{®} erhältlich sind.

Die erfindungsgemäße Vorrichtung kann optional mindestens eine Zuführvorrichtung für COz-haltiges Gas aufweisen. Diese Gas dient dazu, zusätzlich Nährstoff für die in den Biofilmen befindlichen Algen zur Verfügung zu stellen und ist nicht in jedem Falle erforderlich, sondern wird nur angepasst an die jeweiligen genauen Bedingungen eingesetzt (denn es gibt Fälle, in denen kein zusätzliches COz notwendig ist).

Optional kann die erfindungsgemäße Vorrichtung Elektronik- und gegebenenfalls Hydraulik-Betriebseinrichtungen, wie insbesondere Kontroll- und Regeleinrichtungen für Temperatur, Wasserfluss, insbesondere in den Biofilmen, Beleuchtung, COz-Zufuhr, Belüftung Gaszu- und -abfuhr umfassen. Die Kontrolleinrichtungen können dabei zum Beispiel Sensoren umfassen, beispielsweise für pH, Leitfähigkeit etc., wobei dies IoT-Sensoren sein können, um die Daten kontinuierlich und Online verfolgen und überwachen zu können, was dann ein sofortiges Eingreifen (manuell oder automatisiert) ermöglicht, falls dies ausgehend von den Daten notwendig erscheint. Nicht notwendig sind diese Betriebseinrichtungen in der einfachsten Ausführungsform, wenn das Abwasser, über zum Beispiel Rinnen, in die Zuführvorrichtung gegeben wird, das Wasser die Biofilme einfach durchläuft und am Ende einfach abfließt und als Beleuchtung lediglich eine lichtdurchlässige Gehäusewand dient. Üblicherweise jedoch werden Wasserzu- und Abfluss sowie die Durchflussrate durch die Biofilme geregelt oder zumindest eingestellt und überwacht, so dass dann Elektronik- und gegebenenfalls Hydraulik-Betriebseinrichtungen vorhanden sind. Diese können in einem oder mehreren Gehäusen angeordnet sein und können innerhalb oder außerhalb des Gehäuses der erfindungsgemäßen Vorrichtung angeordnet sein. In bevorzugten Varianten der vorliegenden Erfindung sind diese innerhalb angeordnet, um die Transportabilität (und Stapelbarkeit etc.) der erfindungsgemäßen Vorrichtung nicht zu beeinträchtigen.

In manchen bevorzugten Varianten der vorliegenden Erfindung weisen das Gehäuse und/oder die Betriebseinrichtungen Schnittstellen zur optionalen Integration von COz-Zufuhr, der Stromzufuhr von externen Quellen zum Beispiel und bevorzugt Photovoltaik und/oder Windkraft, auf.

In weiteren bevorzugten Varianten der vorliegenden Erfindung weist die erfindungsgemäß Vorrichtung, bzw. insbesondere deren Betriebseinrichtungen, Warnsystem für Pump-, Klimasystem- und/oder LED-Stopp auf.

In bevorzugten Ausgestaltungen der vorliegenden Erfindung ist auf den oberen Flächen der Kultivierungsflächen zunächst ein Aufwuchsmaterial für den Biofilm, bevorzugt Mikroalgen-Biofilm, aufgebracht. Und erst anschließend darauf der jeweilige Biofilm, bevorzugt Mikroalgen-Biofilm. Dieses Aufwuchsmaterial kann in bevorzugten Varianten der vorliegenden Erfindung entweder aus robustem und langlebigem Material, bevorzugt Glasfasergewebe, Polypropylen, Edelstahl, besonders bevorzugt als Netz oder Gewebe, insbesondere mit einer Maschengröße >2, oder kompostierbaren Material, bevorzugt aus Leinen, Hanf, Baumwolle, Sisal, besonders bevorzugt ausgestaltet als Netz oder Gewebe, insbesondere mit einer Maschengröße >2, ausgestaltet sein; bei kurzen Kultivierungszyklen mit Ausrichtung auf Biofilm-/Düngerproduktion, kann dies auch Jute sein, insbesondere ein Netz oder Gewebe, insbesondere mit einer Maschengröße >2, aus Jute.

Wie oben bereits angedeutet ist die vorliegende Erfindung nicht auf eine genaue Anzahl von übereinander angeordneten Kultivierungsflächen beschränkt. Insofern werden in bevorzugten Ausgestaltungen der vorliegenden Erfindung mindestens 3, mindestens 4, mindestens 5, mindestens 6, mindestens 7, mindestens 8, mindestens 9, mindestens 10 oder mehr Kultivierungsflächen übereinander in dem Gehäuse angeordnet. Es ist im Rahmen der vorliegenden Erfindung demgemäß auch möglich zum Beispiel 15 Kultivierungsflächen oder 20 Kultivierungsflächen (sowie entsprechend alle anderen zwischen 10 und 20 liegenden Anzahlen) oder sogar noch mehr übereinander anzuordnen. Dies wird in der Regel nur durch die Praktikabilität eingeschränkt, weil zum Beispiel jeder Container nur ein beschränktes Platzangebot/Volumen aufweist, und in diesem nicht beliebig viele Geräte angeordnet werden können.

In bevorzugten Varianten der vorliegenden Erfindung sind zwei öffen- und schließbare Zugänge vorhanden, die an zwei gegenüberliegenden Seiten des Gehäuses liegen und als Personenzugänge ausgestaltet sind.

In weiteren bevorzugten Varianten der vorliegenden Erfindung sind die Kultivierungsflächen in einer Hälfte des Gehäuses angeordnet und die andere Hälfte des Gehäuses ist als (begehbarer) Wartungsraum bzw. Wartungsbereich konfiguriert, und in der Mitte des Gehäuses sind Lampen angeordnet, die konfiguriert sind, die Kultivierungsflächen und die darauf befindlichen Biofilme zu beleuchten.

In noch weiteren bevorzugten Varianten der vorliegenden Erfindung sind die Zuführvorrichtung zur Aufbringung von aufzuarbeitendem Abwasser (4) und Abführvorrichtung für das aufgearbeitete Wasser (8) mit normgerechten Anschlüssen versehen.

In noch weiteren bevorzugten Varianten der vorliegenden Erfindung sind auf der Oberseite der Vorrichtung ein oder mehrere Photovoltaik-Elemente angeordnet, die dazu konfiguriert sind, die interne Elektronik der Vorrichtung mit Strom zu versorgen. In diesem Fall ist es bevorzugt, wenn die erfindungsgemäße Vorrichtung dann noch Sekundärbatterien umfasst, die von den Photovoltaik-Elementen geladen werden können, wenn ein Stromüberschuss erzeugt wird, und die in der Lage sind, die erfindungsgemäße Vorrichtung mit Strom zu versorgen, wenn die Photovoltaik-Elemente keine Strom erzeugen können (zum Beispiel nachts).

In bevorzugten Ausführungsformen ist die erfindungsgemäße Vorrichtung mit entsprechenden Schnittstellen versehen, die es ermöglichen, den mit der Photovoltaik-Elementen erzeugten Strom an externe Verbraucher oder ein lokales Stromnetz zu liefern, wenn der erzeugt Strom gerade nicht von der erfindungsgemäßen Vorrichtung gebraucht wird.

Die Photovoltaik-Elemente sind also optionale Vorrichtungen, für den Fall, dass ein autarker(er) oder nachhaltiger(er) Betrieb angestrebt wird.

Weitere bevorzugte Varianten der vorliegenden Erfindung zeichnen sich dadurch aus, dass die Aufarbeitung des Wassers über Phytoremediation erfolgt.

In bevorzugten Ausführungsformen umfasst die erfindungsgemäße Vorrichtung mindestens eine integrierte Transportsicherung insbesondere einen Hauptschalter um den Container unter Strom zu setzen, und/oder einen Hauptabsperrhahn um ATS-System mit Abwasser zu versorgen.

In noch weiteren, bevorzugte Varianten der vorliegenden Erfindung weist die Vorrichtung einen Frischwasserzulauf an dem Fußende des Gehäuses auf, insbesondere Innen, der insbesondere zur hygienischen Endreinigung der Gerätschaften und des Personals (Händewaschen) dienen kann.

Das Gehäuse der erfindungsgemäßen Vorrichtung kann in bevorzugten Ausführungsformender vorliegenden Erfindung demgemäß ein ISO-Container, zum Beispiel ein Schiffscontainer, besonders bevorzugt ein 40-Fuß-Container, 20-Fuß-Container oder 10-Fuß-Container sein, insbesondere bevorzugt ist ein 40-Fuß-Container.

In Varianten der vorliegenden Erfindung können die Neigungen der einzelnen Kultivierungsflächen im Bedarfsfalle angepasst werden. Insbesondere können die einzelnen Kultivierungsflächen unabhängig voneinander in ihren Neigungen geändert werden. Die Neigungsveränderung kann entweder manuell oder mechanisch/elektronisch unterstützt erfolgen; für letzteren Zweck sind insbesondere Motoren installiert. Im einem einfachen Falle sind die verschiedenen Kultivierungsflächen an ihrem unteren (niedrigeren) Ende beweglich gelagert und an ihrem oberen Ende mit Rastnasen versehen, die in entsprechende Vertiefungen von Haltestangen eingreifen können (ähnlich wie bei Regalsystemen üblich).

Eine sehr einfache, aber im Rahmen der vorliegende Erfindung mögliche Art und Weise, die übereinander angeordneten Kultivierungsflächen auszuführen, sind Regalsysteme, worin die einzelnen Kultivierungsflächen auf den jeweiligen Regalböden angeordnet werden, oder die Regalböden selbst die Kultivierungsflächen bilden, wobei die Regalböden entsprechend ausgestaltet sein müssen, damit dies funktioniert (insbesondere Metallböden), und die Böden nicht gerade eingesetzt werden, sondern an der einen Seite höher als an der anderen. In dieser Variante muss darauf geachtet werden, dass die oberen Flächen der Regalböden entweder so modifiziert werden, dass sie die Kultivierungsflächen halten können, zum Beispiel durch Klebstoffe oder mechanische Verbindungselemente wie Klemmen oder Schrauben, oder so modifiziert werden, dass sie selbst als Kultivierungsflächen dienen können, also insbesondere die vorder- und Rückseiten mit Rändern versehen sind, die ein Auslaufen des Wassers verhindern.

In manchen bevorzugten Ausführungsformen sind alle vorhandenen Kultivierungsflächen parallel übereinander angeordnet und alle in der gleichen Richtung geneigt und keine Kultivierungsflächen sind in die andere Richtung geneigt.

Im Rahmen der vorliegenden Erfindung können im Prinzip alle denkbaren Abwässer aufbereitet werden, sofern diese nicht toxisch für die verfügbaren Biofilme sind. Dabei können im Rahmen der vorliegenden Erfindung auch Abwässer aufbereitet werden, die für manche Spezies toxisch sind, für andere jedoch nicht - dann würden andere (gegen diese Abwässer tolerante oder resistente) Organismen für den Biofilm verwendet werden. Beispiele für Abwässer, die im Rahmen der vorliegenden Erfindung aufbereitet werden können sind: kommunale Abwässer, Abwässer aus/von Tierhaltung, oder auch Biogasabwässer, industrielle Abwässer aus der Lebensmittelproduktion und -verarbeitung (unkritisch), aus Gesundheitseinrichtungen (kritisch falls mit Pharmazeutika bzw. Pathogenen belastet), aus Textilverarbeitung (kritisch falls noch mit Farbstoffen bzw. Fixierern belastet), aus belastenden Produktionsanlagen (kritisch falls mit Schwermetallen oder PFAS belastet), aus Dekontaminationsmaßnahmen von Sickerwasser aus Deponien und Altlasten (z.B. aus aktivem Abpumpen) - wobei diese Liste selbstverständlich nicht abschließend ist (sein kann). "Kritisch" bezieht sich in diesem Zusammenhang darauf, ob die Abwässer Substanzen mit Gesundheits- oder Umweltrisiken im Sinne der internationalen, nationalen und/oder kommunalen Vorgaben enthalten (z.B. europäische UWWTD, deutsche AbwV).

Ferner sind Gegenstand der vorliegenden Erfindung Verfahren zur Abwasseraufarbeitung, bei denen man Abwasser auf eine Vorrichtung gemäß vorliegender Erfindung aufbringt und das aufgearbeitete Wasser wieder auffängt.

Eine bevorzugte Ausführungsform eines erfindungsgemäßen Verfahrens zur Aufarbeitung von Abwasser unter Einsatz der erfindungsgemäßen Vorrichtung umfasst die folgenden Schritte:
i) Aufstellung der erfindungsgemäßen Vorrichtung, bevorzugt waagerecht (bezogen auf den Gehäuseboden), besonders bevorzugt ebenerdig und/oder witterungsfest;
ii) optional Funktionsprüfung und Re-Justierung von Einzelkomponenten nach der Aufstellung;
iii) Anschluss an die Abwasserquelle, insbesondere die am Ort der Aufstellung befindliche Abwasserquelle, und den Auslass, insbesondere den am Ort der Aufstellung befindlichen Auslass, sofern entsprechende Anschlüsse, insbesondere genormte Anschlüsse, vorhanden sind (ansonsten Verbindung von Zu- und Abfluss ohne Anschlüsse, zum Beispiele nur über Wasserrinnen);
iv) Etablierung einer gleichmäßigen Abwasserverteilung über die gesamte Breite der Kultivierungsflächen durch die Schmutzwasserverteiler;
v) optional Beimpfung des Aufwuchsmaterials mit lokal, das heißt am Ort der Aufstellung oder in der näheren Umgebung (bevorzugt im Umkreis von maximal 2 km) gesammelten natürlichen Mikroalgen;
vi) Etablierung des Mikroalgen-Biofilms und des kontinuierlichen Betriebs mit angepasstem Abwasservolumen bis ein kompakter Bewuchs entstanden ist;
vii) Etablierung des kontinuierlichen Betriebs mit einem Abwasservolumen nach Bedarf und Zielsetzung;
viii) kontinuierliche oder diskontinuierliche Prüfung des Systems durch Kontrollmessungen der Abwasserqualität im Zu- und Ablauf, und ggf. Nachjustierung des Abwasserzulaufs;
ix) manuelle Ernte des Mikroalgen-Biofilms nach Bedarf durch abschaben (insbesondere im Falle von langlebigen Substraten) oder wechseln des Aufwuchsmaterials (insbesondere im Falle von biologisch abbaubarem bzw. kompostierbarem Substrat).

Dabei bedeutet in Schritt i) ebenerdig und/oder witterungsfest eine Aufstellung entsprechend der Klimazone am Aufstellungsort, insbesondere ein solides Fundament sowie ein Gehäuse mit ausreichender Stabilität für eventuelle Schneelasten und/oder gegen Winde (Windfestigkeit) , Vorrichtungen zur Regenableitung, Vorrichtungen zur Beschattung oder das Aufstellen im (teilweisen) Schatten (zu hohe Temperaturen im Inneren fördern die Wasserverdunstung gegebenenfalls über ein akzeptables Maß hinaus).

In bevorzugten Ausführungsformen der vorliegenden Erfindung wird unter einem "kompakten Bewuchs" in Schritt vi) ein durchgehender Bewuchs des Substrates von mindestens 80%, bevorzugt mindestens 90%, besonders bevorzugt mindesten 95%, (bezogen auf die Oberfläche) mit einem Biofilm einer Schichtdicke von 2 mm bis 20 mm, bevorzugt 3 mm bis 15 mm, insbesondere 5 mm bis 10 mm, verstanden. Das "angepasste Abwasservolumen" bedeutet dabei, dass ein gleichmäßiger Wasserfluss eingestellt wird, der weder zu langsam (kein ausreichender Transport der Stoffe) noch zu schnell (das Wasser läuft ohne ausreichende Wechselwirkung ab) ist; dies ist dem Fachmann auf Basis seines allgemeinen Fachwissens ohne weiteres möglich.

In bevorzugten Ausführungsformen der vorliegenden Erfindung wird unter "einem Abwasservolumen nach Bedarf und Zielsetzung" in Schritt vii) entsprechend der Zielstellung entweder eine Anpassung des Abwasservolumens zur optimalen Remediation (z.B. 50-prozentige Reduktion der N oder P-Werte) oder eine Anpassung des Abwasservolumens zur optimalen Biofilmproduktion (z.B. für Biogassubstrat) verstanden. Zielvorgaben können auch den rechtlichen und/oder betrieblichen Vorgaben entsprechen (z.B. europäische Verordnung 2020/741, EU CAP Strategie und Zero Pollution Action Plan).

In bevorzugten Ausführungsformen der vorliegenden Erfindung wird in Schritt ix) zwischen zwei prinzipiellen Ausführungsformen unterschieden:
Im Falle eines langlebigen Substrats (siehe auch oben) wird über lange Zeiträume widerholt abgeerntet (kontinuierlicher Prozess). Insbesondere bei (kritischen) Abwässern und Biofilmen, wo die direkte Kompostierung nicht möglich ist, muss das Substrat möglichst inert gegenüber Kontaminationen und robust sein.
Im Falle von biologisch abbaubarem bzw. kompostierbarem Substrat (siehe auch oben) wird nicht abgeschabt und bis zum Erreichen der Biofilmstärke verwendet. Umwelttoxisch sichere Abwässer können mit diesen Substraten behandelt werden und die Ernte wird mit der Kompostierung beendet.

Nicht zuletzt ist Gegenstand der vorliegenden Erfindung die Verwendung einer Vorrichtung gemäß der vorliegenden Erfindung zur Abwasseraufarbeitung, bevorzugt mittels Phytoremediation.

Die Anordnung von parallelen Stapeln von (statischen) Kultivierungsflächen in der erfindungsgemäßen Vorrichtung ermöglicht eine Reduktion der Aufstellungsfläche (Landverbrauch).

Insbesondere die Anordnung der Kultivierungsflächen in fixierte Neigungswinkel von 2°, wie es eine bevorzugte Variante der vorliegenden Erfindung vorsieht, ermöglicht eine kostengünstige und wartungsarme Konstruktion, weil 2° ein guter Wert ist, bei dem das Wasser langsam und laminar abfließt, um eine gute Nährstoffaufnahme (Kontaktzeit) und ein Biofilmwachstum zu generieren.

In Varianten der vorliegende Erfindung kann mittels einer justierbaren Schmutzwasserpumpe für die Zuführung von aufzureinigendem Abwasser eine Regulation des Abwasserdurchlaufs und der resultierenden Kontaktzeit zwischen Biofilm, insbesondere Mikroalgen-Biofilm, und Abwasser erzielt werden, was eine Anpassung entsprechend des Verschmutzungsgrades (Objektiv Remediation) ermöglicht.

Zudem ermöglicht eine justierbare Schmutzwasserpumpe zur Regulation des Abwasserdurchlaufs eine Anpassung entsprechend des Abwasservolumens.

Insofern umfasst die erfindungsgemäße Vorrichtung in bevorzugten Varianten eine solche justierbare Schmutzwasserpumpe. Diese kann, muss es aber nicht, auch integraler Teil der Abwasserzuführungsvorrichtung sein.

In den Fällen, in denen eine Pumpe für das Abwasser eingesetzt wird, ermöglicht eine solche justierbare Schmutzwasserpumpe (mit Verteiler auf alle Kultivierungsflächen) eine Minimierung des Energieverbrauchs gegenüber Systemen mit mehreren Pumpen.

Die Integration des Mikroalgen-Biofilm-Reaktors (also der Vielzahl von Kultivierungsflächen) in ein allseits umschlossenes Gehäuse, bevorzugt einen Standard Container, mit einem lateralen Fenster und künstlicher LED-Beleuchtung ermöglicht eine Eindämmung von belasteten und/oder potenziell gesundheits- oder umweltschädlichen Abwässern (Pharmazeutika, Schwermetalle, etc.).

Zusätzlich erfolgt im Rahmen der vorliegenden Erfindung Phytoremediation von atmosphärischem COz durch den Algen-Biofilm. In Feldversuchen wurden Algen-Biofilme mit Kohlenstoffgehalten von 20 bis 40%Trockengewicht erzielt. Diese direkte biologische Fixierung von Kohlenstoff (C) qualifiziert die vorliegende Erfindung als Negative Emissions Technology (NET). Je nach Verwertungsform des Algen-Biofilms ist die kategorische Zuordnung zu den Technologien Soil Carbon Sequestation (SCS), Bioenergy Carbon Capture & Storage (BECCS) oder Biochar (BC) möglich.

Besonderer Vorteil der vorliegenden Erfindung, insbesondere für die Fälle, in denen das Gehäuse ein ISO-Container bzw. Standardcontainer ist, sind:
- es wird ein transportfähiges, integrationsfähiges und bedarfsorientiertes System zu Abwasserbehandlung erhalten,
- eine Aufstellung an saisonalen und/ oder entlegenen Punktquellen ist möglich.

Die bevorzugten Ausführungsformen der vorliegenden Erfindung, bei denen das Gehäuse mindestens ein lichtdurchlässiges, bevorzugt tageslichtdurchlässiges, Seitenelementen in einer Gehäusewandung, und innerhalb des Gehäuses angeordnete Lampen, bevorzugt LED aufweist, ermöglicht
i) einen kontinuierlichen Betriebs- und Arbeitsablauf,
ii) eine klima- und witterungsunabhängige Abwasserbehandlung,
iii) eine gleichbleibende Mikroalgen-Biofilm Produktion.

Die im Rahmen der Erfindung mögliche und in einigen Ausführungsformen bevorzugte Zuführung von COz zur Phytoremediation ermöglicht eine Steigerung der Mikroalgen-Biofilm Produktion.

Es ist im Rahmen der vorliegenden Erfindung ebenfalls möglich, eine optionale autarke Energieversorgung zu erreichen, indem ein Auf- und/ oder Anbau von Photovoltaik und Windkraftanlagen an dem Gehäuse (Container) erfolgt.

Eine besonders bevorzugte Vorrichtung gemäß der vorliegenden Erfindung ist eine Vorrichtung zur Aufarbeitung von Abwasser, wobei die Vorrichtung umfasst:
I) ein allseits geschlossenes Gehäuse, das zwei öffen- und schließbare Zugänge aufweist,
II) im Inneren des Gehäuses übereinander, und parallel zueinander angeordnet, zwei oder mehr, insbesondere zehn, Kultivierungsflächen, mit einer Neigung von einer Seite des Gehäuses zur gegenüberliegenden Seite, in Bezug auf die Horizontale von 2°,
III) die oberen Flächen der Kultivierungsflächen belegende Mikroalgen-Biofilme, wobei die auf den unterschiedlichen Kultivierungsflächen aufgebrachten Biofilme alle gleich sind,
IV) eine Zuführvorrichtung zur insbesondere gleichmäßigen Aufbringung von aufzuarbeitendem Abwasser auf alle Kultivierungsflächen, wobei die mindestens eine Zuführvorrichtung so angeordnet ist, dass das aufzuarbeitende Wasser auf das jeweils höhere Ende der jeweiligen Kultivierungsflächen aufgebracht wird, und wobei die Zuführvorrichtung eine regelbare Pumpe umfasst;
V) eine Abführvorrichtungen für das aufgearbeitete Wasser, wobei die mindestens eine Abführvorrichtung so angeordnet ist, dass sie das aufgearbeitete Wasser von dem jeweils niedrigeren Ende der jeweiligen Kultivierungsflächen abführt,
VI) mindestens eine passive Belüftungseinrichtung für das Gehäuse, konfiguriert frische Luft, insbesondere Umgebungsluft, zuzuführen und verbrauchte Luft abzuführen, insbesondere in Form von Belüftungslöchern,
VII) zwei, bevorzugt regelbare, Einrichtungen zur Lichtzufuhr, nämlich
   a lichtdurchlässige, bevorzugt tageslichtdurchlässige, Seitenelementen in einer Gehäusewandung, und
   c innerhalb des Gehäuses angeordnete Lampen, bevorzugt LED;
VIII) eine Zuführvorrichtung für COz-haltiges Gas;
IX) Elektronik- und gegebenenfalls Hydraulik-Betriebseinrichtungen, wie insbesondere Kontroll- und Regeleinrichtungen für Temperatur, Wasserfluss, Beleuchtung, Gaszu- und -abfuhr.

In besonders bevorzugten Ausführungsformen der vorliegenden Erfindung wird das Wasser nicht im Kreislauf geführt. Das heißt, es ist erfindungsgemäß bevorzugt, dass Wasser im einmaligen Durchlauf kontinuierlich zu- und abzuführen.

Die einzelnen Teile der erfindungsgemäßen Produkte, stehen in fachüblicher und bekannter Art und Weise miteinander in Wirkverbindung.

Die vorliegende Erfindung basiert mithin auf anhaftenden (Algen-)Biofilmen, wobei freischwimmende/suspendierte Mikroorganismen (Algen) nicht erwünscht sind (einzelne Mikroorganismen (Algen), die sich von den Filmen ablösen (zum Beispiel aufgrund mechanischer Krafteinwirkung) können vorkommen, sind aber weder gewollt, noch relevant im Vergleich zu dem Gesamtbiofilm). Die Prozessführung erfolgt mit (Ab-)Wasser im kontinuierlichen Betrieb (einmaliger Durchlauf des Wassers). Weiterhin ist der Ressourcenaufwand bei der Ernte vergleichsweise gering (einfaches Abschaben genügt). Und es wird eine Technologie zur Phytoremediation von Abwässern bereitgestellt.

Im Unterschied zu den in D. Reinecke et al., Separation and Purification technology 305 (2023) 122471, zitierten Möglichkeiten bietet die vorliegende Erfindung erhebliche Vorteile. In Reinecke et al. war wesentlich die Kostenreduktion durch reduzierte Aufstellfläche. Das dort verglichene einlagige System ist jedoch ungeeignet für eine gleichbleibende Biofilmausprägung, da ein Nährstoffgefälle zu hoher Variabilität im Biofilm führt (Hunger am Auslass). Die dort ebenfalls verglichenen vertikalen Systeme führen zu Biomasseverlust und sind störungsanfällig.

Das Stapeln von Flowways (Kultivierungsoberflächen) im Sinne der vorliegenden Erfindung ist neu. Das geschlossene und gestapelte ATS-System der vorliegenden Erfindung reduziert den Verdunstungsdruck, erhöht die Reaktorsteuerungsmöglichkeiten und Biosicherheit.

Die verschiedenen Ausgestaltungen der vorliegenden Erfindung, z.B. - aber nicht ausschließlich - diejenigen der verschiedenen abhängigen Ansprüche oder einzelner in den Figuren beschriebenen Ausgestaltungen, können in beliebiger Art und Weise miteinander kombiniert werden, auch wenn diese Kombinationen nicht explizit genannt sind, sofern solche Kombinationen sich nicht widersprechen.

In den Beispielen dargelegte Verfahrensschritte sind als Varianten bevorzugter Ausgestaltungsformen der vorliegenden Erfindung auszulegen.

### Beispiel:

Die Erfindung wird nun unter Bezugnahme auf das folgende, nicht-limitierende Beispiel weiter erläutert.

### Beispiel 1:

Hier wird tabellarisch die Remediationsleistung einer erfindungsgemäßen Vorrichtung dargestellt. Die erfindungsgemäße Vorrichtung war dabei ein 40-Fuß-Container (mit den Dimensionen 12200 mm x 2400 mm x 2600 mm), in dem 10 Kultivierungsflächen parallel und mit gleichem Abstand übereinandergestapelt waren, jeweils mit einer Neigung gegenüber der horizontalen von 2°. Die einzelnen Kultivierungsoberflächen hatten dabei jeweils eine Oberfläche von 11500 mm mal 1500 mm; die Seiten waren im 90°-Winkel hochgebogen (wodurch verhindert wird, dass die Biofilme seitlich von den Flächen rutschen/fallen). Die Wasserhöhe betrug etwa 20 mm. Es ergab sich eine Fläche von 17,25 m² und ein Volumen von 0,35 m³ (pro Kultivierungsoberfläche) und die Fließgeschwindigkeit des Wasser durch die Kultivierungsoberflächen lag bei etwa 20 L/min. Insgesamt ergab sich eine Fläche von 172,5 m² und ein Volumendurchfluss von 200 L/min (entsprechend etwa 288 m³/d). Als Material für die Kultivierungsflächen wurde Edelstahl verwendet. Als Aufwuchsmaterial für den Mikroalgen-Biofilm wurde Glasfasergewebe mit einer Maschengröße >2 verwendet und für den Biofilm Mikroalgen aus der lokalen Umgebung (d.h. vor Ort vorkommende Organismen).

Die resultierende Remediationsleistung wurde über einen Zeitraum von elf Monaten bestimmt. Die gemittelten Daten sind in der folgenden Tabelle 1 wiedergegeben.

**Tabelle 1: Remediationsleistung pro Durchströmung und elementare Zusammensetzung des Algen-Biofilms (jährliches Mittel, 03.2022 bis 04.2023).**

| | Abwasser | | Biofilm | |
|---|---|---|---|---|
| | [% Liter] | Standardabweichung | [% Biomasse] | Standardabweichung |
| N | -15,6 | 8,7 | 2,0 | 0,283 |
| P | -16,9 | 17,2 | 7,7 | 0,845 |
| K | -3,4 | 7,3 | 0,3 | 0,039 |
| C | | | 18,5 | 1,560 |
| Rest | | | 71,5 | |

Eine Bestimmung über einen Zeitraum von 24 Monaten ergab die in Tabelle 2 wiedergegebenen Ergebnisse:

**Tabelle 2:**

| | Abwasser | | Biofilm | |
|---|---|---|---|---|
| | [% Liter] | StandardAbweichung | [% Biomasse] | StandardAbweichung |
| N | -37 | 10% | 3,5 | 1,2% |
| P | -35 | 25% | 4,4 | 2,7% |
| K | -3 | 7% | 0,6 | 0,4% |
| C | | | 23,4 | 5,5% |

### Figuren:

Die im Folgenden mit Bezugnahme auf die Figuren näher erläuterten Ausführungsformen der vorliegenden Erfindung stellen verschiedene bevorzugte Ausführungsformen dar, viele der im Folgenden in einzelnen Figuren dargestellten Merkmale bzw. Ausführungsformen sind mit in anderen Figuren oder der übrigen Beschreibung dargestellten Merkmalen und Ausführungsformen kombinierbar, insbesondere wo die Merkmale entsprechend beschrieben sind. Die Figuren sind überdies nicht limitierend auszulegen und nicht maßstabsgetreu. Weiterhin enthalten die Figuren nicht alle Merkmale, die übliche Vorrichtungen/Anlagen aufweisen, sondern sind auf die für die vorliegende Erfindung und ihr Verständnis wesentlichen Merkmale reduziert, beispielsweise sind Schrauben, Schläuche, Halterungen etc. nicht oder nicht im Detail dargestellt.

Gleiche Bezugszeichen/-ziffern bedeuten dabei gleiche oder gleichwirkende Vorrichtungsteile.

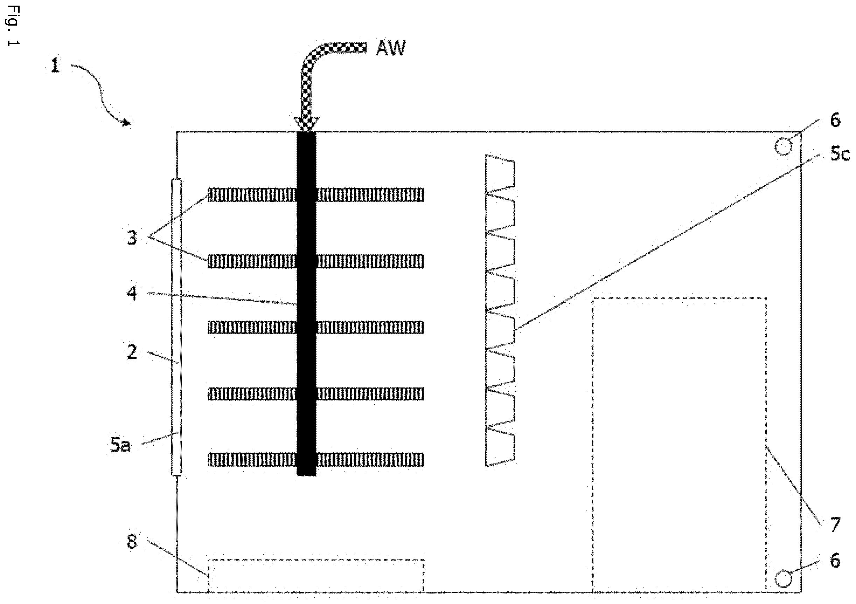

Figur 1 zeigt eine Frontalansicht einer erfindungsgemäßen Vorrichtung **1,** das heißt eine Kopfansicht auf das Gehäuse **2,** das in bevorzugten Ausgestaltungen der Erfindung aus einem Schiffscontainer, insbesondere einem 40-Fuß-Schiffscontainer (mit den Dimensionen 12200 mm x 2400 mm x 2600 mm), gebildet wird. Es ist hier dargestellt, dass eine Seite (im Bild links dargestellt) zum Teil aus einem transparenten Material, welches für Tageslicht durchlässig ist, gebildet wird. Dieses lichtdurchlässige Seitenelement **5a** ist normalerweise als eines oder mehrere Fenster ausgestaltet; es ist aber auch möglich eine größere lichtdurchlässige Platte, zum Beispiel aus Plexiglas, zu verwenden. Es ist der Einfachheit halber in dieser Figur nicht dargestellt, jedoch können alle Seiten des Gehäuses 2 solche lichtdurchlässigen Elemente **5a** aufweisen. Genauso können in der Gehäusedecke lichtdurchlässige Deckenelemente **5b** vorhanden sein. In Figur 1 ist eine Variante der vorliegenden Erfindung gezeigt, bei der die Kultivierungsflächen **3** innerhalb des Gehäuses **2** nur in einer Hälfte angeordnet sind (hier links). Zwar nehmen die Kultivierungsflächen **3** in dieser Figur nur etwa ein Drittel der Breite ein (was rein illustrativ ist), jedoch können die Kultivierungsflächen **3** natürlich auch mehr oder weniger Breite als in der Figur gezeigt, oder sogar die ganze Breite (was aber oft nicht gewünscht ist, da dann kein einfacher Zugang in das Gehäuse **2** bzw. zu den Kultivierungsflächen **3** mehr gewährleistet ist; insofern wird in der Regel zumindest ein geringer Teil der Breite als Durchgang/Zugang freibleiben) einnehmen. Auch ist die vorliegende Erfindung nicht auf die gezeigten fünf übereinander angeordneten Kultivierungsflächen **3** beschränkt, es können selbstverständlich mehr oder weniger sein. In der linken Hälfte ist zudem eine Zuführvorrichtung für aufzuarbeitendes Wasser **4** gezeigt, mittels derer aufzuarbeitendes Wasser (Abwasser) **AW** auf die einzelnen Kultivierungsflächen **3** verteilt wird. aufzuarbeitende Wasser (Abwasser) **AW** wird von außen zugeführt, hier mittels geflecktem Pfeil illustriert, zum Beispiel über einen geeigneten Abwasserzulauf wie etwa einen Schlauch oder auch eine Rinne (nicht gezeigt). Ebenfalls nicht gezeigt ist die Möglichkeit, das aufzuarbeitende Wasser (Abwasser) **AW** mittels einer (Schmutz-)Pumpe, die (außen) an dem Kopfende des Gehäuses (Containers) angeordnet sein kann in den Einlass der Zuführvorrichtung **4** zu leiten. In einigen bevorzugten Varianten der vorliegenden Erfindung weist das Gehäuse Belüftungseinrichtungen auf, die hier als Belüftungslöcher **6** gezeigt sind, auf; statt einfacher Löcher können aber auch mechanisch und/oder elektronisch unterstützte Belüftungseinrichtungen vorhanden sein. Idealerweise (für eine bessere Luftzirkulation) befinden sich diese an oder auf der Unter- und Oberseite des Gehäuses **2.** Etwa in der Mitte des Inneren des Gehäuses **2** sind mit den Lampen **5c** weitere Einrichtungen zur Lichtzufuhr gezeigt; dies können insbesondere LED-Lampen sein, die zum Beispiel lateral aufgehängt sein können, was in der Figurenperspektive nicht erkennbar ist. Ferner ist in der Figur mittels gestrichelter Linie eine Tür angedeutet, als eine bevorzugte Ausführungsform eines öffen- und schließbaren Zugangs **7.** Ferner ist mittels gestrichelter Linien noch eine Abführvorrichtung für aufgearbeitetes Wasser **8** angedeutet, die sich am anderen Ende des Gehäuses befindet.

Es ist zu beachten, dass in der Figur 1 das Fenster und ebenfalls die Kultivierungsfläche links dargestellt ist. Dies ist zwar bevorzugt aber nicht zwingend immer der Fall - denn es ist bevorzugt, wenn die Kultivierungsfläche direkt neben dem (einem) Fenster angeordnet ist, damit die Kultivierungsflächen, und damit die Biofilme, ausreichend Licht bekommen; davon kann aber abgewichen werden, insbesondere, wenn Lampen 5c und/oder lichtdurchlässige Elemente 5b vorhanden sind.

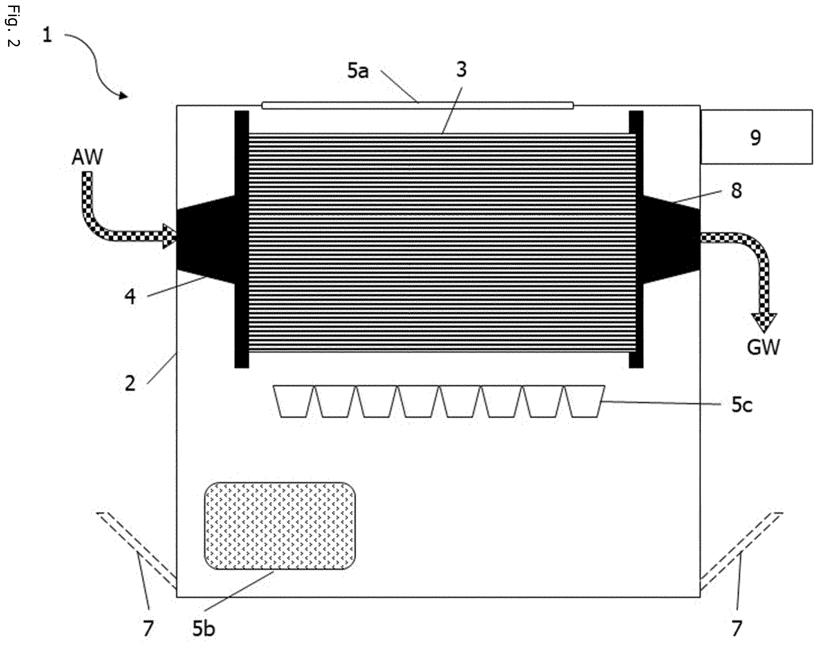

Figur 2 zeigt eine Aufsicht des gleichen Gehäuses **2** wie Figur 1. Gezeigt ist hier die gleiche Zuführvorrichtung für aufzuarbeitendes Wasser **4** mit Zufluss von aufzuarbeitendem Wasser **AW,** wie in Figur 1 und am anderen Ende der Kultivierungsflächen **3** (von denen aufgrund der perspektive nur das oberste zu sehen ist) noch die Abführvorrichtung für aufgearbeitetes Wasser **8.** Das gereinigte Wasser **GW** wird dann in manchen Varianten nach außen abgeführt (hier durch gefleckten Pfeil dargestellt). Es ist aber auch möglich das gereinigte Wasser im Kreis zu führen und nochmals den Kultivierungsflächen **3** aufzugeben, um eine weitere Aufreinigung zu ermöglichen; in der Regel ist dies aber nicht der Fall. Ebenso ist es möglich, das gereinigte Wasser in einem Sammelbehältnis bis zur Freigabe zwischenzulagern, zum Beispiel bis es geprüft und entsprechend der rechtlichen oder betrieblichen Vorgaben freigegeben wurde. Die Lampen **5c** sind in dieser Darstellung in ihrer lateralen Anordnung zu erkennen, genauso wie das lichtdurchlässige Seitenelement **5a.** Zusätzlich zu diesen beiden ist in Figur 2 noch ein, optionales, lichtdurchlässiges Deckenelement **5b** dargestellt (von denen eines, mehrere oder auch gar keine vorhanden sein können). Ähnlich wie in Figur 1 sind mittels gestrichelter Linie öffen- und schließbare Zugänge **7** angedeutet. Elektronik-/Hydraulik-Betriebseinrichtungen **9** sind in Figur 2 extern angeordnet gezeigt, in einigen bevorzugten Varianten der vorliegenden Erfindung sind diese aber innerhalb des Gehäuses angeordnet, da diese Variante die Transportabilität der Gesamtvorrichtung **1** vereinfacht.

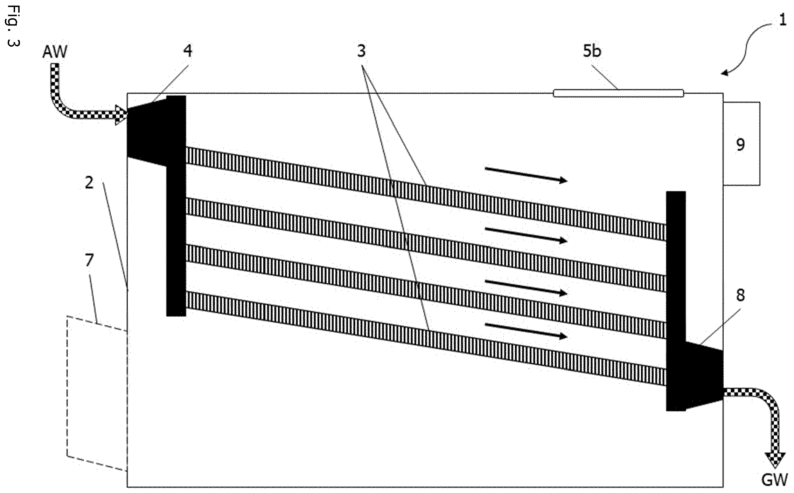

Figur 3 zeigt schließlich eine Seitenansicht der gleichen Vorrichtung **1.** Der besseren Übersichtlichkeit halber sind hier jedoch nur vier Kultivierungsflächen **3** übereinander dargestellt. Die in Figur 3 dargestellten Neigungswinkel der Kultivierungsflächen **3** sind zur besseren Illustration derart dargestellt und entsprechen nicht unbedingt den in Realität anzuwendenden Winkeln. Die vier über den jeweiligen Kultivierungsflächen **3** angeordneten Pfeile geben die Flussrichtung des aufzuarbeitenden Wassers über die Kultivierungsflächen **3** bzw. die darauf befindlichen (Mikroalgen-)Biofilme an. Wie in Figur 2 auch sind hier die Zuführvorrichtung für aufzuarbeitendes Wasser **4** mit Zufluss von aufzuarbeitendem Wasser **AW,** und am anderen Ende der Kultivierungsflächen **3** noch die Abführvorrichtung für aufgearbeitetes Wasser **8** gezeigt, sowie dass in der gezeigten Variante das gereinigte Wasser **GW** nach außen abgeleitet wird. Der Wassereinlass der Zuführvorrichtung **4** ist höher angeordnet als die oberste Kultivierungsfläche **3** und der Wasserauslass der Abführvorrichtung **8** ist niedriger als die unterste Kultivierungsflächen **3;** auf diese Art und Weise erfolgt der Wassertransport bzw. der Wasserfluss entsprechend der Schwerkraft und erfordert an sich keine zusätzliche energieaufwändige Hilfe von zum Beispiel Pumpen. In dieser Figur sind somit die Zu- und Abführvorrichtung für das Wasser einerseits als Verteiler ausgestaltet und andererseits als Sammler. Es ist im Rahmen der vorliegenden Erfindung aber auch möglich, jeweils mehrere Zu- und Abführvorrichtungen vorzusehen, zum Beispiel für jeweils einige Kultivierungsflächen und/oder für einzelne Kultivierungsflächen; je nach Bedarf.

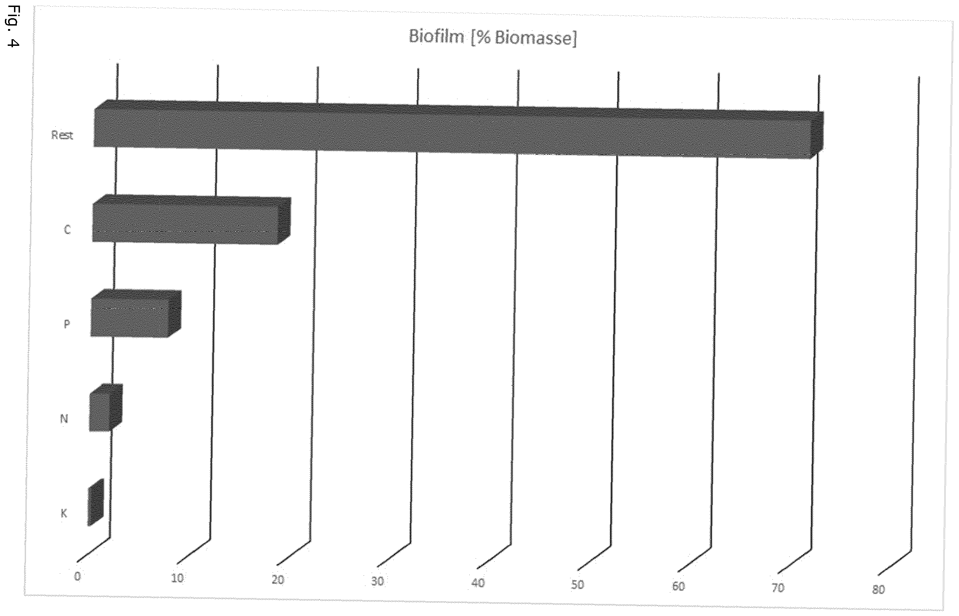

Figur 4 zeigt die Daten der Tabelle 1 als Diagramm, wobei der prozentuale Anteil der verschiedenen Elemente (K, N, P, C und Rest) an der Gesamtmasse aufgetragen ist.

### Bezuaszeichenliste:

- 1: Vorrichtung zur Aufarbeitung von Abwasser
- 2: Gehäuse
- 3: Kultivierungsflächen
- 4: Zuführvorrichtung für aufzuarbeitendes Wasser
- 5: Einrichtung zur Lichtzufuhr
- 5a: lichtdurchlässiges Seitenelement
- 5b: lichtdurchlässiges Deckenelement
- 5c: Lampen
- 6: Belüftungseinrichtung
- 7: öffen- und schließbarer Zugang
- 8: Abführvorrichtung für aufgearbeitetes Wasser
- 9: Elektronik-/Hydraulik-Betriebseinrichtungen
- AW: aufzuarbeitendes Wasser (Abwasser)
- GW: gereinigtes Wasser
- Pfeile: Flussrichtung (von AW, GW bzw. generell)

## Patentansprüche

1. Vorrichtung zur Aufarbeitung von Abwasser, wobei die Vorrichtung umfasst:
I) ein allseits geschlossenes Gehäuse, das ein oder mehr öffen- und schließbare Zugänge aufweist;
II) im Inneren des Gehäuses übereinander angeordnet zwei oder mehr Kultivierungsflächen, mit einer jeweiligen Neigung in Bezug auf die Horizontale zwischen 0.5° und 7,5°,
III) die oberen Flächen der Kultivierungsflächen belegende Biofilme, wobei die auf den unterschiedlichen Kultivierungsflächen aufgebrachten Biofilme alle gleich, zum Teil gleich oder alle unterschiedlich sind;
IV) mindestens eine Zuführvorrichtung zur Aufbringung von aufzuarbeitendem Abwasser auf ein oder mehrere Kultivierungsflächen, wobei die mindestens eine Zuführvorrichtung so angeordnet ist, dass das aufzuarbeitende Wasser auf das jeweils höhere Ende der jeweiligen Kultivierungsflächen aufgebracht wird;
V) mindestens eine Abführvorrichtungen für das aufgearbeitete Wasser, wobei die mindestens eine Abführvorrichtung so angeordnet ist, dass sie das aufgearbeitete Wasser von dem jeweils niedrigeren Ende der jeweiligen Kultivierungsflächen abführt;
VI) mindestens eine Belüftungseinrichtung für das Gehäuse, konfiguriert, Luft zuzuführen und verbrauchte Luft abzuführen;
VII) mindestens eine Einrichtung zur Zufuhr von Licht zu den Biofilmen;
VIII) optional mindestens eine Zuführvorrichtung für COz-haltiges Gas;
IX) optional Elektronik- und gegebenenfalls Hydraulik-Betriebseinrichtungen.

2. Vorrichtung (1) nach Anspruch 1, **dadurch gekennzeichnet, dass** auf den oberen Flächen der Kultivierungsflächen zunächst Aufwuchsmaterial für den Biofilm, bevorzugt Mikroalgen-Biofilm, entweder aus robustem und langlebigen Material, bevorzugt Glasfasergewebe, Polypropylen, Edelstahl, besonders bevorzugt als Netz oder Gewebe, insbesondere mit einer Maschengröße >2, oder kompostierbaren Material, bevorzugt aus Leinen, Hanf, Baumwolle, Sisal oder Jute, besonders bevorzugt als Netz oder Gewebe, insbesondere mit einer Maschengröße >2, aufgebracht ist und anschließend darauf der jeweilige Biofilm, bevorzugt Mikroalgen-Biofilm.

3. Vorrichtung (1) nach einem der vorhergehenden Ansprüche, **dadurch gekennzeichnet, dass** das Gehäuse ein quaderförmiges Gehäuse ist, bevorzugt ein, gegebenenfalls umgebauter, Container, besonders bevorzugt ein, gegebenenfalls umgebauter, Standardcontainer, insbesondere ein, gegebenenfalls umgebauter, 10-Fuß-Schiffscontainer, 20-Fuß-Schiffscontainer oder 40-Fuß-Schiffscontainer.

4. Vorrichtung (1) nach einem der vorhergehenden Ansprüche, **dadurch gekennzeichnet, dass** mindestens 3, 4, 5, 6, 7, 8, 9, 10 oder mehr Kultivierungsflächen übereinander in dem Gehäuse angeordnet sind.

5. Vorrichtung (1) nach einem der vorhergehenden Ansprüche, **dadurch gekennzeichnet, dass** zwei öffen- und schließbare Zugänge vorhanden sind, die an zwei gegenüberliegenden Seiten des Gehäuses liegen und als Personenzugänge ausgestaltet sind.

6. Vorrichtung (1) nach einem der vorhergehenden Ansprüche, **dadurch gekennzeichnet, dass** die Kultivierungsflächen (3) in einer Hälfte des Gehäuses angeordnet sind, die andere Hälfte des Gehäuses als Wartungsbereich konfiguriert ist, und in der Mitte des Gehäuses als Einrichtung zur Zufuhr von Licht Lampen angeordnet sind, die konfiguriert sind, die Kultivierungsflächen (3) und die darauf befindlichen Biofilme zu beleuchten.

7. Vorrichtung (1) nach einem der vorhergehenden Ansprüche, **dadurch gekennzeichnet, dass** die Zuführvorrichtung zur Aufbringung von aufzuarbeitendem Abwasser (4) und die Abführvorrichtung für das aufgearbeitete Wasser (8) mit normgerechten Anschlüssen versehen ist.

8. Vorrichtung (1) nach einem der vorhergehenden Ansprüche, **dadurch gekennzeichnet, dass** auf der Oberseite der Vorrichtung ein oder mehrere Photovoltaik-Elemente angeordnet sind, konfiguriert, die interne Elektronik der Vorrichtung mit Strom zu versorgen.

9. Vorrichtung (1) nach einem der vorhergehenden Ansprüche, **dadurch gekennzeichnet, dass** die Aufarbeitung des Wassers über Phytoremediation erfolgt.

10. Vorrichtung (1) nach einem der vorhergehenden Ansprüche, insbesondere nach Anspruch 6, **dadurch gekennzeichnet, dass** als eine Einrichtung zur Zufuhr von Licht mindestens eine Lampe mit mehreren Beleuchtungsmitteln oder mehrere Lampen vorgesehen sind, die von der Gehäusedecke herabhängen und so befestigt sind, dass sie einzeln oder in Gruppen verschoben werden können, insbesondere seitlich.

11. Verfahren zur Abwasseraufarbeitung, **dadurch gekennzeichnet, dass** man Abwasser auf eine Vorrichtung gemäß einem der Ansprüche 1 bis 10 aufbringt und das aufgearbeitete Wasser wieder auffängt.

12. Verfahren zur Abwasseraufarbeitung, insbesondere nach Anspruch 11, umfassend die folgenden Schritte:
i) Aufstellung einer Vorrichtung gemäß einem der Ansprüche 1 bis 10;
ii) optional Funktionsprüfung und Re-Justierung von Einzelkomponenten nach der Aufstellung;
iii) Anschluss an die Abwasserquelle und den Auslass, sofern entsprechende Anschlüsse vorhanden sind;
iv) Etablierung einer gleichmäßigen Abwasserverteilung über die gesamte Breite der Kultivierungsflächen durch die Schmutzwasserverteiler;
v) optional Beimpfung von Aufwuchsmaterial der Kultivierungsflächen mit lokal gesammelten Mikroalgen;
vi) Etablierung eines Mikroalgen-Biofilms und des kontinuierlichen Betriebs mit angepasstem Abwasservolumen bis ein kompakter Bewuchs entstanden ist;
vii) Etablierung des kontinuierlichen Betriebs mit einem Abwasservolumen nach Bedarf und Zielsetzung;
viii) kontinuierliche oder diskontinuierliche Prüfung des Systems durch Kontrollmessungen der Abwasserqualität im Zu- und Ablauf, und ggf. Nachjustierung des Abwasserzulaufs;
ix) manuelle Ernte des Mikroalgen-Biofilms nach Bedarf durch abschaben oder wechseln des Aufwuchsmaterials.

13. Verwendung einer Vorrichtung gemäß einem der Ansprüche 1 bis 10 zur Abwasseraufarbeitung, bevorzugt mittels Phytoremediation.
